(19) Europäisches Patentamt
European Patent Office
Office européen des brevets

(11) Publication number: **0 326 417 A2**

(12) # EUROPEAN PATENT APPLICATION

(21) Application number: **89300826.8**

(22) Date of filing: **27.01.89**

(51) Int. Cl.4: **C 07 H 19/01**
A 61 K 31/70

(30) Priority: **27.01.88 JP 16360/88**

(43) Date of publication of application:
**02.08.89 Bulletin 89/31**

(84) Designated Contracting States:
**AT BE CH DE ES FR GB GR IT LI LU NL SE**

(71) Applicant: **SHIONOGI SEIYAKU KABUSHIKI KAISHA trading under the name of SHIONOGI & CO. LTD.
12, 3-chome, Dosho-machi Higashi-ku
Osaka (JP)**

(72) Inventor: **Kondo, Eiji
4-22-18, Ishibashi
Ikeda-shi Osaka (JP)**

**Hayashi, Yoshiyuki
585-5, Nomura-cho
Kusatsu-shi Shiga (JP)**

**Mitsugi, Takashi
2-15-27, Kitatoyonaka-cho
Izumiotsu-shi Osaka (JP)**

**Konishi, Takao
10-11, Tateishi-cho
Ikeda-shi Osaka (JP)**

(74) Representative: **Bizley, Richard Edward et al
BOULT, WADE & TENNANT 27 Furnival Street
London EC4A 1PQ (GB)**

(54) **Antibiotic k-41 Derivatives, their preparation and use.**

(57) New polyether-type antibiotic K-41 derivatives of the formula

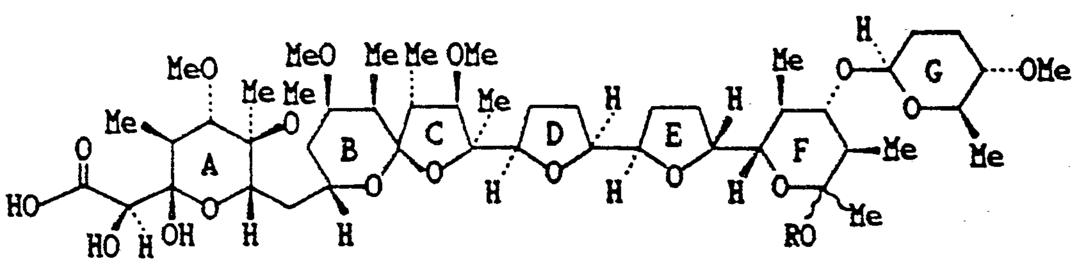

wherein Me is methyl, R is substituted lower alkyl, and the wavy lines indicate the α- or β-configuration or a mixture thereof, and their salts, are useful as anticoccidial agents.

EP 0 326 417 A2

**Description**

## ANTIBIOTIC K-41 DERIVATIVES, THEIR PREPARATION AND USE

The present invention relates to novel derivatives of a polyether-type antibiotic, K-41. In more detail, it relates to therapeutic materials, particularly compounds which have anticoccidial activity and which are useful in the veterinary field as anticoccidial agents.

The antibiotic K-41 is a polyether-type antibiotic produced by Streptomyces hygroscopicus K-41 which was deposited with Fermentation Research Institute, Agency of Industrial Science and Technology (1-3, Higashi 1 chome Tsukuba-shi ibaraki-ken 305, JAPAN) on February 7, 1972 as FERM P-No. 1342, then transferred on May 1, 1981 as FERM BP-1648. Its physiological properties and the process for production thereof are disclosed in Japan Kokoku No. 52-21077 and J. Antibiotics, 29, 10-14 (1976). The structure elucidated by X-ray analysis is disclosed in J.C.S. Chem. Comm. 682-683, (1978). Moreover, K-41 is expected to be an active ingredient for acaricides or anticoccidial agents (JPN, Kokai Nos. 53-20420 and 55-27115) because of its acaricidial and anticoccidial activities. The antibiotic K-41 has some problems, however, such as a liability to be oxidized or to accumulate in poultry when administrated or like disadvantages. In order to solve such problems, derivatives such as K-41-C(2) esters (JPN Kokai 57-7492), K-41-C(29) ethers (JPN Kokai 57-14597) and the like have been provided.

Various K-41 derivatives have been prepared so far. However, they still have some problems, such as accumulation in poultry. Consequently, suitable derivatives as anticoccidial agents have not yet been developed.

This invention provides antibiotic K-41 derivatives, having anticoccidial activity, of the formula (1):

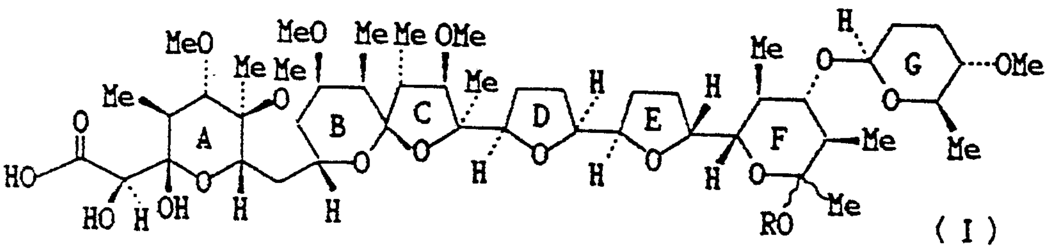

( I )

wherein Me is methyl, R is substituted lower alkyl, and the wavy lines indicate the α- or β-configuration or a mixture thereof, and their salts.

The following preferred explanations are given for various terms used throughout this specification.

The term "lower alkyl" preferably refers to $C_1$ to $C_5$ alkyl, e.g., methyl, ethyl, propyl, isopropyl, butyl, tert-butyl, pentyl, or the like, but it may include $C_6$, $C_7$, or even $C_8$.

In the term "substituted lower alkyl", the substituent may be e.g. a 5-membered oxygen atom-containing heterocycle, aryl, substituted alkoxy or hydroxy. The term "5-membered oxygen atom-containing heterocycle" refers to, for example, 2-tetrahydrofuryl, 3-tetrahydrofuryl, 2-furyl, 3-furyl, or the like.

The term "aryl" refers to e.g. phenyl, naphthyl, or the like.

The term "alkoxy" refers to e.g. $C_1$ to $C_5$ alkyloxy, e.g. methoxy, ethoxy, propoxy, isopropoxy, butoxy, tert-butoxy, pentoxy, or the like.

The substituent bound to alkoxy may be, for example, hydroxyalkoxy, hydroxy or the like.

Thus, the term "substituted alkoxy" may refer to hydroxyalkoxy, e.g. hydroxymethoxy, 2-hydroxyethoxy, 3-hydroxypropoxy, 4-hydroxybutoxy, or the like, or hydroxyalkoxyalkoxy, e.g. 2-(hydroxymethoxy)ethoxy, 2-(2-hydroxyethoxy)ethyoxy, 3-(2-hydroxyethoxy)propoxy, 3-(3-hydroxypropoxy)propoxy, 3-(3-hydroxybutoxy)ethoxy, or the like.

Preferred "substituted lower alkyl" groups include furylalkyl, hydroxyalkyl, hydroxyalkoxyalkyl, hydroxyalkoxyalkoxy, and so forth, e.g. 2-furfuryl, 3-furfuryl, 2-(2-furyl)ethyl, 2-(3-furyl)ethyl, or the like; benzyl, 2-phenylethyl, or the like; hydroxymethyl, 2- hydroxyethyl, 3-hydroxypropyl, 4-hydroxybutyl, or the like; 2-hydroxyethoxymethyl 2-(2-hydroxyethoxy)ethyl, 3-(2-hydroxyethoxy)propyl, 4-(3-hydroxypropoxy) butyl, or the like; and 2-(2-hydroxyethoxy)ethoxy-methyl,, 2-[2-(2-hydroxyethoxy)ethoxy]ethyl, 3-[2-(2-hydroxyethoxy)-ethoxy]propyl, 4-[3-(2-hydroxyethoxy)propoxy]butyl, or the like.

Especially preferred "substituted lower alkyl" groups include 2-furfuryl, benzyl, 2-hydoxyethyl, 2-(2-hydroxyethoxy)ethyl, and 2-[2-(2-hydroxyethoxy)ethoxy]ethyl, and so forth.

Each ring in the structure is named A, B, C, D, E, F, and G, respectively, as a matter of convenience.

The invention also includes a process for preparing a compound as defined above or a salt thereof.

The C(29)-hydroxy of the formula (1) can be easily etherified by conventional etherification e.g.(1) an alcohol may be allowed to react with a halide, (2) an alcohol may be allowed to react with an ester compound, (3) dehydration of alcohol, or the like.

The present invention is explained in more detail by the following Reference Example and Examples, which

are not intended to limit the scope of the invention. In addition, in the accompanying drawings: -

Figures Nos. 1 to 5 show the IR spectra in chloroform of sodium salts of K-41-C(29) furfuryl ether, benzyl ether, 2-drydroxyethyl ether, 2-(2-hydroxyethoxy)ethyl ether, and 2-[2-(2-hydroxyethoxy)-ethoxy]ethyl ether, respectively.

Reference Example

Preparation of sodium salt of K-41 II (R is replaced by hydrogen in formula 1).

*Streptomyces hygroscopicus* K-41 is inoculated into a nutrient medium (pH 7.0) composed of 2.0 % of soluble starch, 1.0 % of Bacto-Soytone, 0.5 % of glycerol, 0.5 % of corn steep liquor, 0.35 % of sodium chloride, 0.3 % of glucose, and 0.5 % of calcium carbonate and cultivated at 27 °C for 48 hours under shaking. The resulting broth is used as inoculum.

The inoculum is transferred into 100 ml of the above nutrient medium which is placed in 500 ml Sakaguchi flask. The cultivation is performed at 27 °C fo 96 hours under shaking. Anti-foaming agent such as P-2000 or lard oil may be added, if necessary.

The cultured broth (2.5 ℓ) thus prepared is filtered to separate mycelium. The mycelium and the filtrate are extracted with 1 ℓ of ethyl acetate, respectively. Each extract is combined and evaporated under reduced pressure to give about 1.5 g of the residue which is dissolved in about 2 ml of chloroform. The solution is applied to column chromatography on 30 g of silica gel (3 x 15 cm) eluted with about 500 ml of chloroform and then which chloroform containing 2 % methanol. The fractions containing K-41 are collected and concentrated to give about 370 mg of the crude product which is crystallized from petroleum ether to give a mixture of potassium salt and sodium salt of K-41. The mixture is dissolved in chloroform. The resulting mixture is shaken with 2 % aqueous solution of tartaric acid to give the free acid. The chlorofrom layer is washed with water and then shaken with 5 % aqueous solution of sodium carbonate to give sodium salt of K-41. The organic layer is evaporated under reduced pressure and the residue is recrystallized from petroleum ether to give about 250 mg of sodium salt of K-41 II as crystals.

Molecular formula: $C_{43}H_{81}O_{18}Na{\bullet}H_2O$ (X-ray analysis)
$^{13}C$-NMRδ ($CDCl_3$): 3.33, 3.36, 3.40, 3.46, 3.53.
m.p. 196 ~ 198 °C
Specific rotation: $[\alpha]_D$ $+1.9 \pm 0.4°$ (23°C, c 1.017, methanol)

Example 1

Preparation of sodium salt of K-41-C(29) furfurylether II (R is 2-furfuryl in formula I)

A solution of 5.0 g of sodium salt of K-41 II in 170 ml of chloroform washed with 2 % aqueous solution of tartaric acid is filtered through absorbent cotton and dried. To the filtrate is added 3.0 g of furfuryl alcohol and then chloroform is evaporated at 55 °C under reduced pressure. The residue is dissolved in additional 9.0 g of furfuryl alcohol and the mixture is heated at 58 °C for 4.5 hours. The reaction mixture is diluted with 200 ml of chloroform and the mixture is washed with 5 % aqueous solution of sodium bicarbonate and then with water, and dried. Chloroform is evaporated to give an oil containing remaining alcohol. The oil dissolved in chloroform is passed through a column on 350 g of Kiesegel 60 (Merck) to remove the remaining alcohol. The ether-compound is eluted with chloroform containing 10 % acetone to give 4.31 g of residue, which is dissolved in 200 ml of chloroform. The mixture is washed with 2 % aqueous solution of tartaric acid, water, 5 % aqueous solution of sodium bicarbonate and then water, dried, and evaporated. The residue dissolved in ethyl ether is crystallized from petroleum ether to give 3.46 g of crude crystals, which is recrystallized from the same solvent system to give 2.76 g of sodium salt of K-41-C(29)-furfuryl ether.
m.p.184.5 ~ 186.5° C

Anal. Calcd. (%) for $C_{53}H_{85}O_{19}Na$:

| | | | | | | |
|---|---|---|---|---|---|---|
| | C | 60.67; | H | 8.17; | Na | 2.19; |
| Found (%) | C | 60.91; | H | 8.43; | Na | 1.95. |

IR spectrum: See Fig. 1.IRνmax ($CHCl_3$) 3419, 1612 $cm^{-1}$.

Example 2

Preparation of sodium salt of K-41-C(29) benzyl ether 12 (R is benzyl in formula I)

A solution of 2.0 g of K-41 sodium salt II in 100 ml of chloroform washed with 2 % aqueous solution of tartaric acid is filtered through absorbent cotton and dried. The filtrate is evaporated at 55 °C under reduced pressure. To the residue is added 3.0 g of benzyl alcohol and the mixture is heated at 58 to 60 °C for 3 hours. The reaction mixture is diluted with 100 ml of chloroform, washed with 5 % aqueous solution of sodium bicarbonate and water, and concentrated under reduced pressure. The concentrate is applied to column chromatography on Kiesegel 60 (Merck) to separate an ether-compound mixture from remaining benzyl alcohol. The resulting

ether-compound mixture is applied to column chlormatography (Lobar column 310 x 25, Merck) eluted with ethyl acetate to give 1.65 g of the ether-compound, which is dissolved in 100 ml of chloroform. The mixture is washed with 2 % aqueous solution of tartaric acid, water, then 5 % aqueous solution of sodium bicarbonate and water, dried, and evaporated. The residue is crystallized from ethyl ether-petroleum ether to give 0.963 g of sodium salt of K-41-C(29)-benzyl ether 12 as colorless crystals.
Mp.199.5 ~ 201° C (colored)
IR spectrum: See Fig. 2.
IRν max($CHCl_3$): 3418, 1614 $cm^{-1}$.

Example 3

Preparation of sodium salt of K-41-C(29) 2-hydroxyethyl ether 13 (R is 2-hydroxyethyl in formula I)

A solution of 6.0 g of sodium salt of K-41 II in 160 ml of chloroform washed with 2 % tartaric acid is filtered through absorbent cotton and dried. To the mixture is added 4.5 g of ethylene glycol and chloroform is evaporated at 55 ° C under reduced pressure. To the residue is added additional 9.0 g of ethylene glycol and 15.0 ml of acetone and the mixture is refluxed at 38 - 39° C for 2.5 hours. Acetone is evaporated at 55 ° C under reduced pressure. The residue dissolved in 200 ml of chloroform is washed with 5 % aqueous solution of sodium bicarbonate and water, dried, and evaporated to give 7.03 g of oil. The oil is purified by column chromatography (Lobar column 440 x 37, Merck) eluted with ethyl acetate to give 4.22 g of ether-compound, which then is dissolved in 200 ml of chloroform. The mixture is treated by the same procedure as described in Example 2, crystallized, and recrystallized to give 3.87 g of sodium salt of K-41-C(29) 2-hydroxyethyl ether 13.
(The crude crystals: 4.01 g)
m.p. 180.5 ~ 182.5° C

Anal. Calcd. (%) for $C_{50}H_{86}O_{19}Na$:

| | | | | | | |
|---|---|---|---|---|---|---|
| | C | 59.27; | H | 8.47; | Na | 2.27; |
| Found (%) | C | 59.31; | H | 8.50; | Na | 1.99. |

IR spectrum: See Fig. 3IRν max($CHCl_3$) 3390, 1611 $cm^{-1}$.

Example 4

Preparation of sodium salt of K-41-C(29) 2-(2-hydroxyethoxy)ethyl ether 14 (R is 2-(2-hydroxyethoxy)ethyl)

A solution of 6.0 g of K-41 sodium salt II in 160 ml of chloroform washed with 2 % aqueous solution of tartaric acid is filtered through absorbent cotton and dried. To the filtrate is added 7.5 g of diethylene glycol and chloroform is evaporated at 55 °C under reduced pressure. To the residue is added additional 7.5 g of diethylene glycol and the mixture is heated at 58 to 59 ° C fo 2.5 hours. The reaction mixture is diluted with 200 ml of chloroform, washed with 5 % aqueous solution of sodium bicarbonate and water, and dried. Chloroform is evaporated. The residue is applied to column chromatography (Lobar column 440 x 37 cm, Merck) eluted with ethyl acetate to give 5.18 g of the ether- compound, which is treated by the same procedure as described in Example 2, crystallized, and recrstallized from ethyl ether-petroleum ether to give 3.26 g of sodium salt of K-41-C(29) 2-(2-hydroxyethoxy)ethyl ether 14.
m.p. 142 ~ 142.5 °C.

Anal. Calcd. (%) for $C_{52}H_{89}O_{20}Na$:

| | | | | | | |
|---|---|---|---|---|---|---|
| | C | 59.07; | H | 8.49; | Na | 2.7; |
| Found (%) | C | 59.31; | H | 8.57; | Na | 2.09. |

I.R. spectrum: See Fig. 4.I.R. ν max ($CHCl_3$) 3410, 1614 $cm^{-1}$.

Example 5

Preparation of sodium salt of K-41-C(29) 2-[2-(2-hydroxyethoxy)ethoxy]ethyl ether 15 (R is 2-[2-(2-hydroxyethoxy)ethoxy]ethyl in formula I)

A solution of 1.67 g of K-41 sodium salt II in 80 ml of chloroform washed with 2 % aqueous solution of tartaric acid is filtered through absorbent cotton and dried. To the filtrate is added 2.58 g of triethylene glycol and chloroform is evaporated at 55 ° C under reduced pressure. The residue is heated at 55 ° C for 10 hours and the mixture is diluted with 100 ml of chloroform. The resulting mixture is washed with 5% aqueous solution of sodium bicarbonate and water, dried, and evaporated at 55° C under reduced pressure. The residue is crystallized from ethyl ether-petroleum ether to give 1.4666 of crude crystals which is recrystallized from the same solvent system to give 1.147 g of sodium salt of K-41-C(29) 2-[2-(2-hydroxyethoxy)ethoxy]ethyl ether 15.
m.p. 179-181 °C.

Anal. Calcd. (%) for $C_{54}H_{93}O_{21}Na$:

| | | | | | | |
|---|---|---|---|---|---|---|
| | C | 58.89; | H | 8.51; | Na | 2.09; |
| Found (%) | C | 59.08; | H | 8.60; | Na | 1.89. |

Experiment (Anticoccidial activity)

a. Effect against Eimeria tenella

Test method: Chickens of 8 days old broilers Arbor acrs (5 chickens per group) were orally inoculated with sporulated oocysts of Eimeria tenella at 5 x $10^4$ per chick. The test compound was administrated to the chickens together with standard feed for infant chickens (made by Nihon Haigo Shiryo) continually until the end of the test (for 8 days after the infection).

As clinical symptoms, the degree of bloody feces during the test, the survival rate, and the oocyst number were observed. On the 8th day from the infection, the body weight was determined and lesions in the caecum were observed upon autopsy.

Notes:

(1) The degree of bloody feces was classified in 4 steps: 0 to 3.
(2) Survival rate: Number of surviving chickens/number of test chickens x 100 (%)
(3) Oocyst number: Count of oocysts existing in 1 g of feces (OPG value)
(4) Caecal lesion score: The degrees of lesion in the caecum were classified to 0 - 4 and the score was calculated as per 10 chickens.

Table 1

| Compd. No.* | Dose (ppm) | Relative weight gain (%) -1~8th day | Bloody feces 4, 5, 6, 7th (day) | | | | Survival rate (%) | Oocyst number (7th day) | Caecal lesion score (8th day) |
|---|---|---|---|---|---|---|---|---|---|
| I 1 | 150 | 99.2 | 0 | 0 | 0 | 0 | 100 | $6.3 \times 10^3$ | 2 |
| | 100 | 90.8 | 0 | 1 | 2 | 1 | 100 | $1.5 \times 10^5$ | 14 |
| | 50 | 64.7 | 1 | 3 | 3 | 2 | 80 | $9.0 \times 10^5$ | 40 |
| I 2 | 150 | 94.7 | 0 | 1 | 1 | 0 | 100 | $8.8 \times 10^4$ | 12 |
| | 100 | 83.3 | 0 | 2~3 | 2 | 1 | 80 | $2.8 \times 10^5$ | 28 |
| | 50 | 75.2 | 1 | 3 | 3 | 1 | 40 | $8.3 \times 10^5$ | 36 |
| II | 150 | 74.0 | 0 | 0 | 0 | 0 | 100 | 0 | 2 |
| | 100 | 101.5 | 0 | 0 | 0 | 0 | 100 | $6.3 \times 10^3$ | 4 |
| | 50 | 98.1 | 0 | 1 | 2 | 1 | 100 | $3.3 \times 10^5$ | 24 |
| Infected Control | 0 | 68.5 | 1 | 3 | 3 | 1 | 80 | $4.3 \times 10^5$ | 38 |
| Uninfected control | 0 | 100.0 | 0 | 0 | 0 | 0 | 100 | 0 | 0 |

*The compound number corresponds to the reference Example or Example.

Table 2

| Compd. No.* | Dose (ppm) | Relative weight gain (%) -1 ~ 8th day | Bloody feces 4, 5, 6, 7th (day) | | | | Survival rate (%) | Oocyst number (7th day) | Caecal lesion score (8th day) |
|---|---|---|---|---|---|---|---|---|---|
| I 3 | 150 | 92.4 | 0 | 0 | 0 | 0 | 100 | $6.3 \times 10^3$ | 0 |
| | 100 | 98.9 | 0 | 1 ~ 2 | 1 | 0 | 100 | $4.4 \times 10^4$ | 8 |
| | 50 | 70.1 | 1 | 3 | 2 | 0 | 60 | $1.4 \times 10^6$ | 38 |
| I 4 | 150 | 93.2 | 0 | 0 | 1 | 0 | 100 | $1.9 \times 10^4$ | 8 |
| | 100 | 91.3 | 0 | 1 | 1 | 0 | 100 | $1.4 \times 10^5$ | 16 |
| | 50 | 64.0 | 1 | 3 | 2 | 0 ~ 1 | 60 | $8.4 \times 10^5$ | 40 |
| I 5 | 150 | 93.2 | 0 | 0 | 0 ~ 1 | 0 | 100 | $3.1 \times 10^4$ | 10 |
| | 100 | 79.8 | 0 | 2 | 2 | 1 | 100 | $6.9 \times 10^4$ | 34 |
| | 50 | 60.8 | 1 | 3 | 2 | 0 ~ 1 | 80 | $6.0 \times 10^5$ | 40 |
| II | 150 | 78.3 | 0 | 0 | 0 | 0 | 100 | 0 | 0 |
| | 100 | 99.6 | 0 | 1 | 1 | 0 | 100 | $1.9 \times 10^4$ | 4 |
| | 50 | 84.0 | 0 ~ 1 | 2 | 2 | 0 | 100 | $7.5 \times 10^5$ | 30 |
| Infected Control | 0 | 59.7 | 2 | 3 | 2 | 0 ~ 1 | 100 | $8.7 \times 10^5$ | 38 |
| Uninfected Control | 0 | 100.0 | 0 | 0 | 0 | 0 | 100 | 0 | 0 |

*The compound number corresponds to the reference Example or Example.

The K-41 derivatives of the present invention have an anticoccidial activity as mentioned above.

The present invention includes, in various aspects, the following:-

(a) the use of a compound as defined above or a salt as defined above in the preparation of a medicament;

(b) a method for making a therapeutic formulation which comprises forumlating for therapeutic use a compound as defined above or a salt as defined above, optionally together with an acceptable diluent, carrier or exupient and/or in unit dosage form;

(c) a therapeutic formulation comprising a compound as defined above or a salt as defined above formulated for therapeutic use, optionally together with an acceptable diluent, carrier or exupient and/or in unit dosage form;

(d) A veterinary formulation comprising a compound as defined above or a salt as defined above formulated for anticoccidial use.

The compounds of the present invention and their pharmaceutically or veterinary acceptable salts (e.g. sodium, potassium, calcium, magnesium, iron, and like salts) may be used solely or in combination e.g. as active ingredients of anticoccidial agents. They may be formulated solely or together with conventional carriers into various dosage forms, e.g. powders, granules, solutions, suspensions, premixed molding compounds, capsules, emulsions, tablets, and so on, and if necessary, disintegrators, lubricants, stabilizers, flavorings agents, moistening agents, coloring agents, preservatives, aromatics, and so on, may also be added. As carriers, those which are conventionally used in agents for poultry can be used, for example, water, lactose, sucrose, talc, colloidal silica, soybean cake, starch, yeast, wheat, defatted rice bran, defatted soybean, corn, wheat bran, and other commercially available feeds for poultry may also be employed.

The present preparations of anticoccidial agents may be given mixed with poultry feed and drinking water, or directly administered orally. Solutions, suspensions, and emulsions are convenient formulations for adding to feed and water, while capsules and tablets are suitable for direct oral administration. In mixing with feed, the present K-41 derivatives may be added in an amount of 10 to 500 ppm, 5 to 300 ppm generally being used when mixed with water. In oral administration, they are generally given at a dose of 20 to 200 mg/kg body weight.

The preparations of the present invention can be administered together with veterinary agents such as known anticoccidial agents and parasite expellents.

As clearly shown by the above results, the compounds of the present invention have anticoccidial activity. Moreover, the disadvantage of K-41, i.e. a tendency to remain in the body, is improved, and it is expected that the safety of the compounds is increased.

## Claims

1. A compound of the formula:

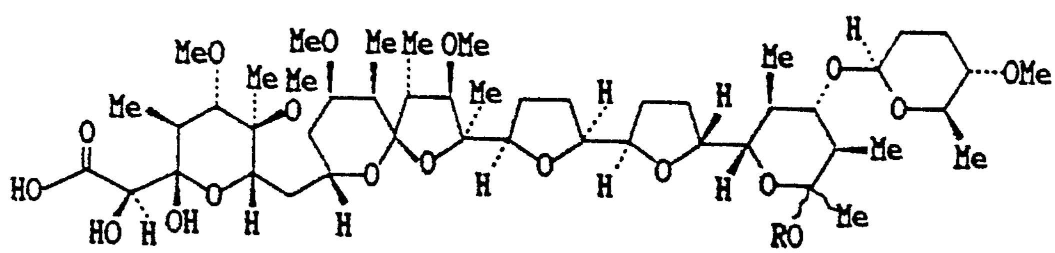

wherein Me is methyl, R is substitured lower alkyl, and the wavy lines represent the α- or β-configuration or a mixture thereof.

2. A compound as claimed in Claim 1, wherein the substituent for lower alkyl is a 5-membered heterocycle, aryl, substituted alkoxy, or hydroxy.

3. A compound as claimed in Claim 1 wherein R is 2-furfuryl, benzyl, 2-hydoxyethyl, 2-(2-hydroxyethoxy)ethyl, or 2-[2-(2-hydroxyethoxy)ethoxy]ethyl

4. A compound as claimed in Claim 1 or Claim 2 wherein lower alkyl is $C_{1-5}$ alkyl.

5. A salt of a compound as claimed in any one of claims 1 to 4.

6. A process for preparing a compound as defined in claim 1 or a salt thereof, which process comprises etherifying a starting compound of the formula

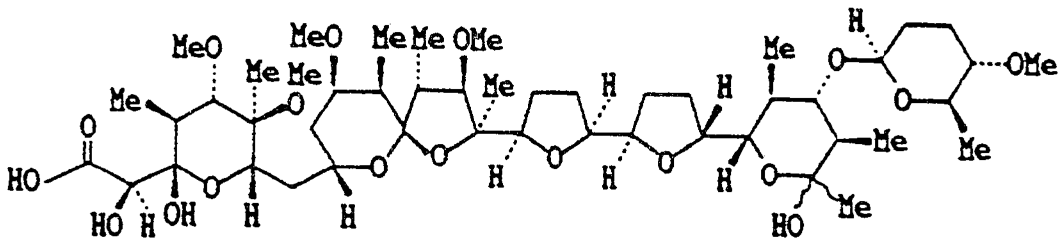

or a salt thereof, e.g. by treating the said starting compound or salt with an alcohol of the formula R-OH, or a reactive derivative thereof.

7. The use of a compound as defined in any one of claims 1 to 4 or a salt as defined in claim 5 in the preparation of a medicament.

8. A method for making a therapeutic formulation which comprises formulating for therapeutic use a compound as defined in any one of claims 1 to 4 or a salt as defined in claim 5, optionally together with an acceptable diluent, carrier or exipient and/or in unit dosage form.

9. A therapeutic formulation comprising a compound as defined in any one of claims 1 to 4 or a salt as defined in claim 5 formulated for therapeutic use, optionally together with an acceptable diluent, carrier or exupient and/or in unit dosage form.

10. A veterinary formulation comprising a compound as defined in any one of claims 1 to 4 or a salt as defined in claim 5 formulated for anticoccidial use.

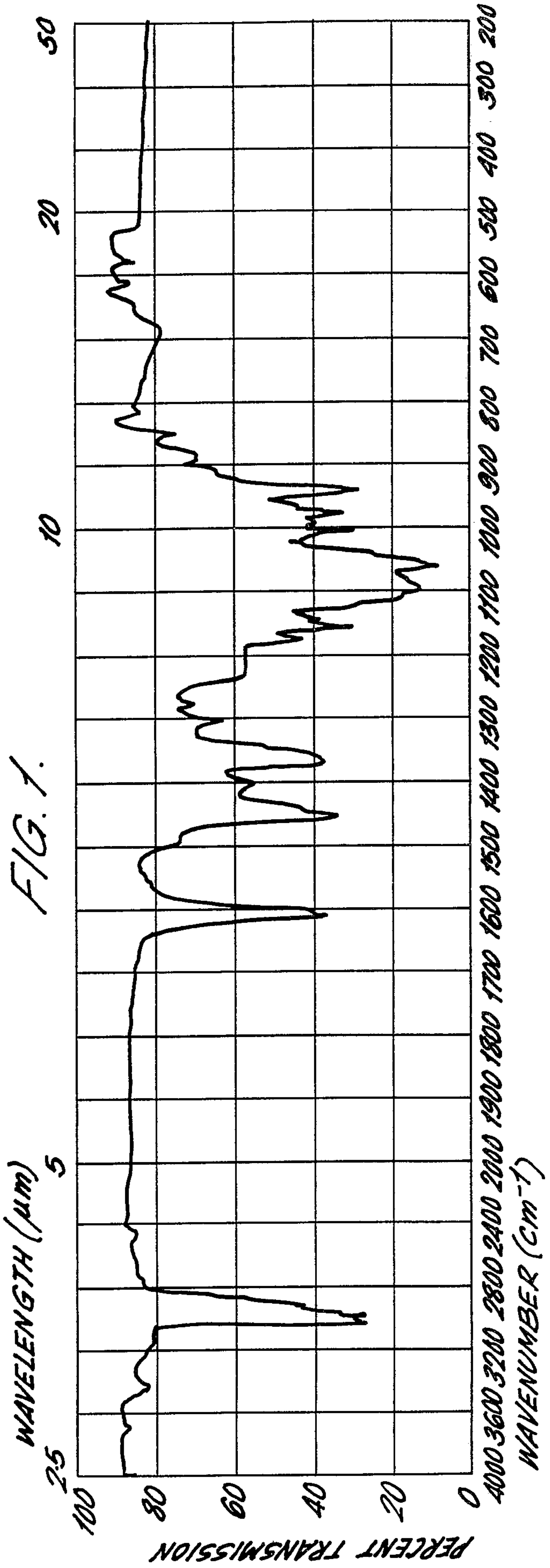
WAVELENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVENUMBER (cm⁻¹)

FIG. 1.

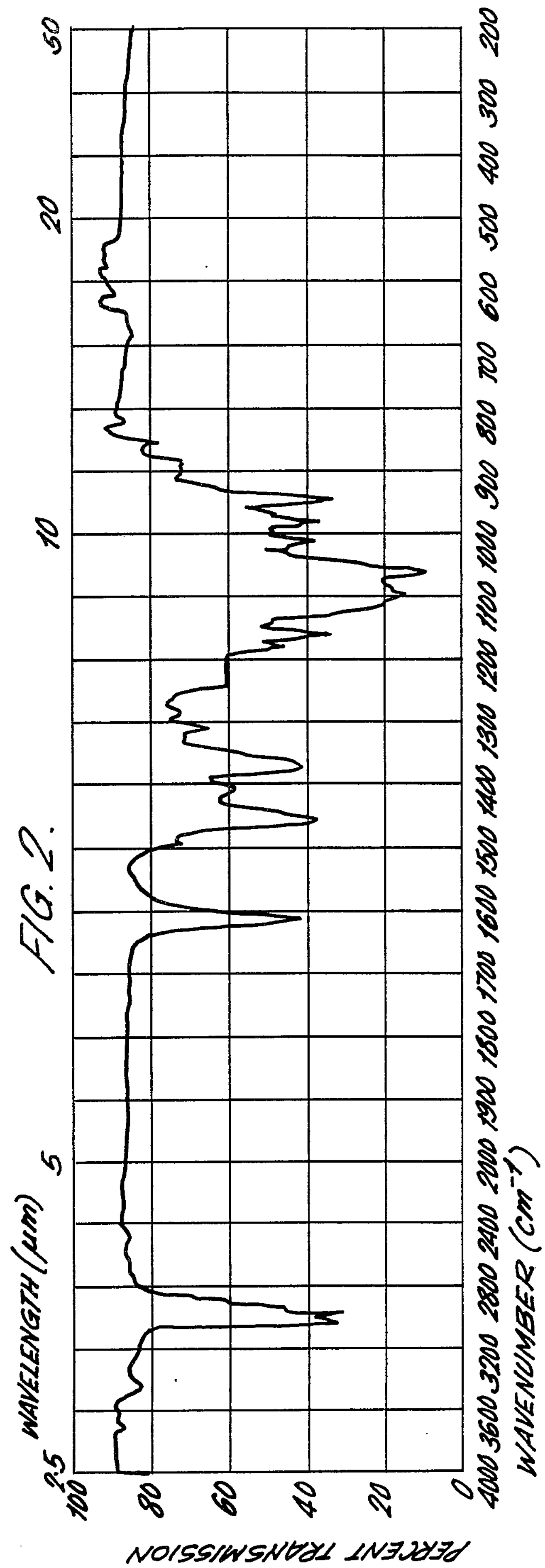
WAVELENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVENUMBER (cm⁻¹)

FIG. 2.

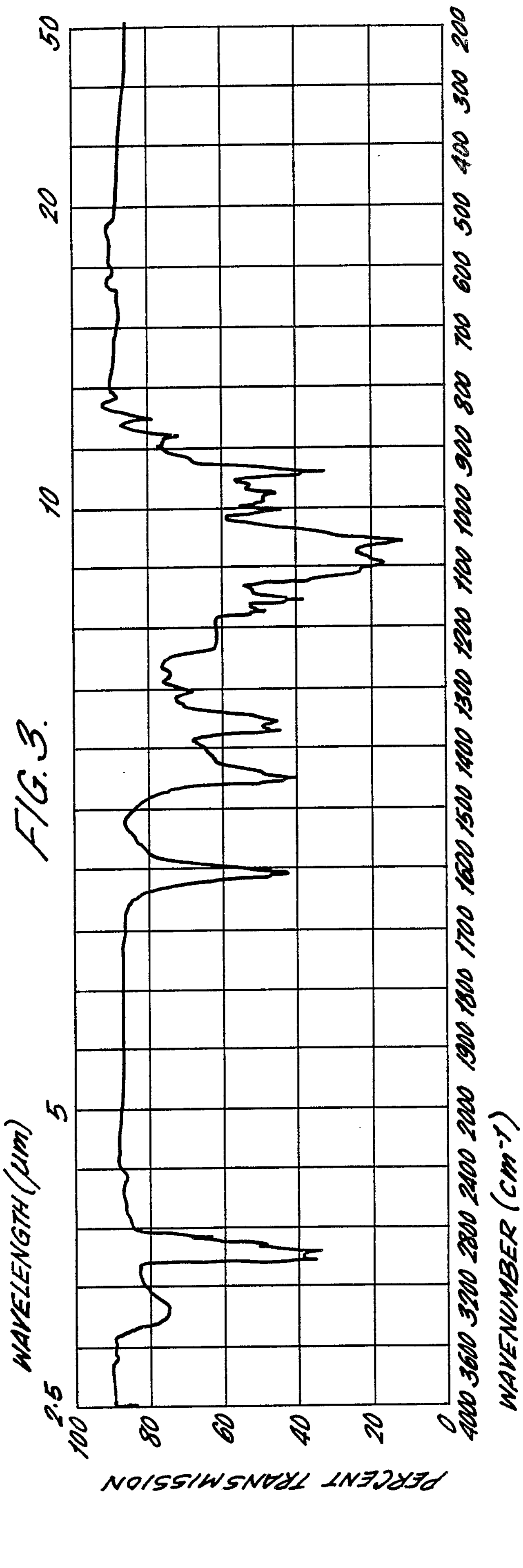
WAVELENGTH (µm)
2.5
5
10
20
50
WAVENUMBER ($cm^{-1}$)
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0

FIG. 3.

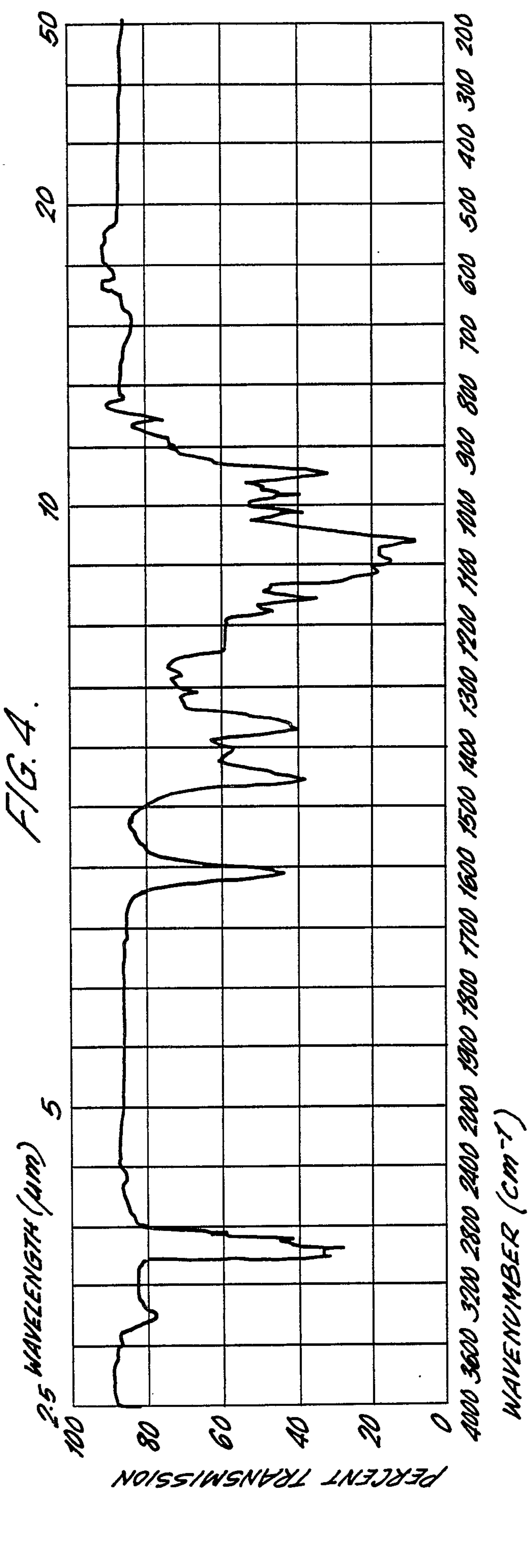
WAVELENGTH (µm)
2.5
5
10
20
50
WAVENUMBER ($cm^{-1}$)
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0

FIG. 4.

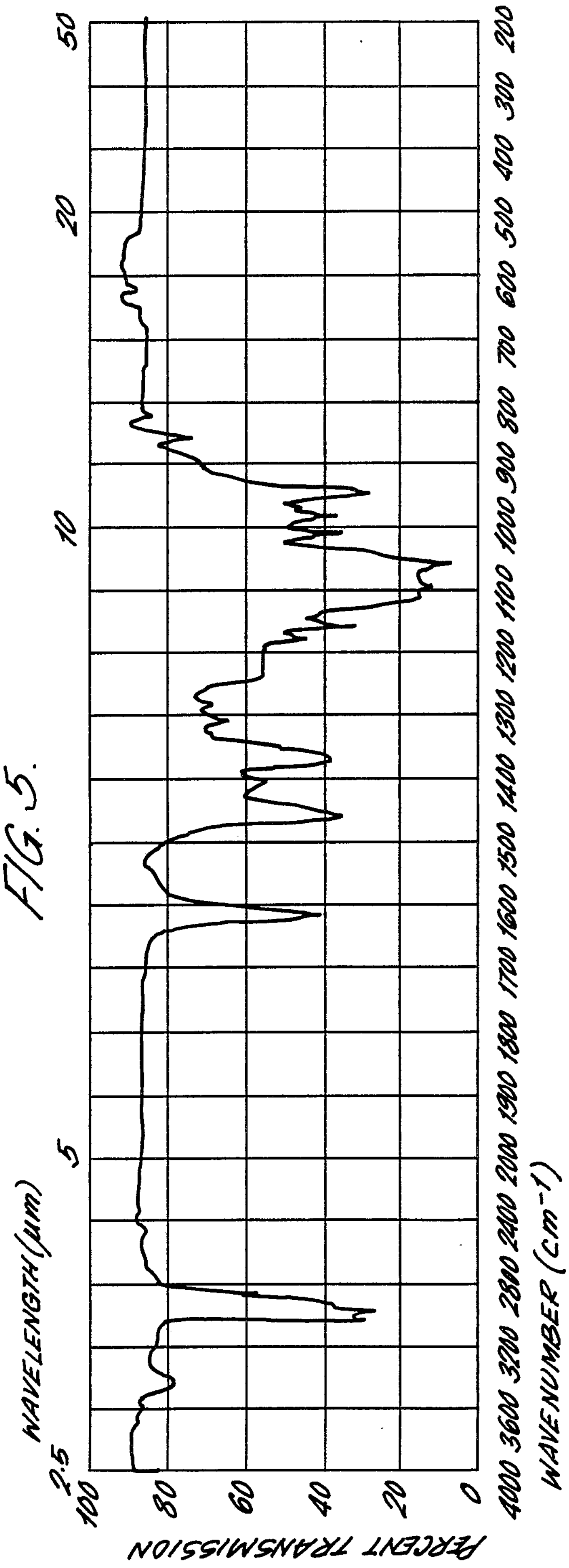
WAVELENGTH (µm)
2.5
5
10
20
50
PERCENT TRANSMISSION
100
80
60
40
20
0
WAVENUMBER (cm⁻¹)
4000
3600
3200
2800
2400
2000
1900
1800
1700
1600
1500
1400
1300
1200
1100
1000
900
800
700
600
500
400
300
200

FIG. 5.